(19) Europäisches Patentamt
European Patent Office
Office européen des brevets

(11) Numéro de publication: 0 102 300
A1

# (12) DEMANDE DE BREVET EUROPEEN

(21) Numéro de dépôt: 83401704.8

(22) Date de dépôt: 24.08.83

(51) Int. Cl.³: C 01 B 17/05
B 01 D 53/34, C 10 K 1/16

(30) Priorité: 25.08.82 FR 8214592

(43) Date de publication de la demande:
07.03.84 Bulletin 84/10

(84) Etats contractants désignés:
BE DE FR GB IT NL

(71) Demandeur: COMPAGNIE FRANCAISE DE RAFFINAGE
Société anonyme dite:
5, rue Michel-Ange
F-75781 Paris Cedex 16(FR)

(72) Inventeur: Biedermann, Jean-Michel
10 rue du Languedoc
F-76290 Montivilliers(FR)

(72) Inventeur: Patureaux, Thierry
3 rue du 11 Septembre 1944 Fontaine la Mallet
F-76290 Montivilliers(FR)

(72) Inventeur: Rossarie, Jean
3 rue Marceau Flandre
F-76000 Le Havre(FR)

(74) Mandataire: Jolly, Jean-Pierre et al,
Cabinet BROT et JOLLY 83, rue d'Amsterdam
F-75008 Paris(FR)

## (54) Procédé d'élimination de l'hydrogène sulfuré contenu dans un mélange gazeux.

(57) L'invention concerne un procédé d'élimination de l'hydrogène sulfuré contenu dans un gaz à épurer, ce procédé étant du type comprenant:

*a* une étape de réaction de l'hydrogène sulfuré avec de l'anhydride sulfureux dans un solvant commun;

*b* une étape d'absorption de l'excès d'hydrogène sulfuré contenu dans l'effluent gazeux de l'étape *a*.

Selon l'invention, l'étape *a* est précédée d'une absorption de l'hydrogène sulfuré du gaz à épurer dans un solvant identique à celui des étapes *a* et *b* et la solution obtenue est utilisée pour la réaction de l'étape *a*.

EP 0 102 300 A1

Procédé d'élimination de l'hydrogène sulfuré contenu dans un mélange gazeux

La présente invention concerne un procédé d'élimination de l'hydrogène sulfuré contenu dans un mélange gazeux.

De nombreux mélanges gazeux, naturels ou provenant d'unités industrielles, contiennent de l'hydrogène sulfuré, qu'il est nécessaire d'éliminer de ces mélanges préalablement à leur utilisation.

Ces mélanges gazeux seront désignés dans la suite de la présente description par l'expression "gaz à épurer", l'expression "gaz épuré" désignant un mélange gazeux d'où l'hydrogène sulfuré a été éliminé de façon pratiquement complète.

Le gaz à épurer peut être, par exemple, le gaz naturel ou l'effluent gazeux d'une unité de raffinage du pétrole brut, telle qu'une unité de désulfuration.

Il est connu, pour éliminer l'hydrogène sulfuré contenu dans le gaz à épurer, d'utiliser des procédés consistant à laver ce gaz par des absorbants, comme, par exemple, les amines. L'hydrogène sulfuré est ensuite restitué par chauffage de la solution, puis converti en soufre dans une unité Claus, où l'hydrogène sulfuré réagit sur l'anhydride sulfureux suivant la réaction:

$$2H_2S + SO_2 \longrightarrow 3S + 2\ H_2O$$

Dans les unités Claus comprenant généralement trois étages, l'anhydride sulfureux est produit, par oxydation de l'hydrogène sulfuré, dans un étage thermique utilisant une température élevée, de l'ordre de 1 400°C, la réaction de Claus ayant lieu dans deux étages catalytiques, où la température est de l'ordre de 200 à 250°C.

Le gaz recueilli à la sortie de l'unité Claus contient encore une faible proportion d'hydrogène sulfuré et d'anhydride sulfureux, qu'il est nécessaire d'éliminer avant le rejet du gaz à l'atmosphère, pour éviter la pollution atmosphérique. Les procédés utilisant une unité Claus ont donc l'inconvénient de nécessiter une unité dite de traitement du gaz de queue. Outre cet inconvénient, il est à noter également que l'étape de restitution de l'hydrogène sulfuré est une grosse consommatrice de calories. De plus, le gaz entrant dans l'étage thermique ne doit pas contenir d'hydrocarbures, d'une part, pour éviter la formation d'oxysulfure de carbone, qui est polluant pour l'atmosphère et n'est généralement pas éliminé dans les étages catalytiques, et, d'autre part, pour éviter la formation de carbone, qui conduit à la production d'un soufre de couleur noire, indésirable.

Il a été proposé d'autres procédés, qui consistent à faire réagir dans une enceinte l'anhydride sulfureux et l'hydrogène sulfuré dans un solvant de ces deux composés, et à recueillir à la sortie de l'enceinte, d'une part, le gaz épuré et, d'autre part, le soufre formé lors de la réaction. Un tel procédé est par exemple décrit dans le brevet français 1 492 013, où le solvant utilisé est un ester neutre de l'acide phosphorique.

Toutefois, afin d'obtenir une élimination aussi complète que possible de l'hydrogène sulfuré, il est nécessaire d'employer une enceinte réactionnelle de très grande taille, ce qui diminue l'intérêt économique d'un tel procédé. Une solution consiste alors à effectuer la réaction de l'anhydride sulfureux et de l'hydrogène sulfuré avec un excédent d'hydrogène sulfuré favorable à la cinétique de la réaction ainsi qu'à sa sélectivité, puis à absorber dans un agent absorbant l'excédent d'hydrogène sulfuré contenu dans le gaz sortant de l'enceinte. La solution d'hydrogène sulfuré dans cet agent absorbant est ensuite

traitée pour restituer l'hydrogène sulfuré, qui est brûlé pour obtenir l'anhydride sulfureux nécessaire à la réaction. Un tel procédé, décrit dans le brevet français n° 2 187 680, a l'inconvénient de nécessiter lui aussi une étape de restitution de l'hydrogène sulfuré qui, comme indiqué précédemment, est une grosse consommatrice de calories. Il a en outre le désavantage de nécessiter l'emploi de deux solvants, ce qui complique la mise en oeuvre du procédé.

La Demanderesse a mis au point un procédé d'élimination de l'hydrogène sulfuré permettant de pallier les inconvénients précités, procédé qui a fait l'objet du brevet français n° 2 411 082. Ce procédé permet d'obtenir un gaz épuré contenant très peu d'hydrogène sulfuré, de l'ordre de 1 000 p.p.m en volume. Il convient tout à fait aux besoins d'une raffinerie qui est la propre consommatrice de son gaz combustible et pour lequel il n'existe pas de spécification sur la teneur en hydrogène sulfuré.

Cependant, les spécifications en matière d'épuration de gaz hydrocarbonés peuvent être plus sévères:

- 4 p.p.m au maximum, pour un gaz de champ devant être transporté dans une canalisation ou devant être liquéfié,
- 0,1 p.p.m au maximum, pour un effluent de gazéification.

Il est en outre peu prudent de traiter directement un gaz soumis à une spécification sévère et impérative dans une unité complexe, susceptible d'un arrêt accidentel, telle qu'une unité mettant en oeuvre la réaction de Claus.

De plus, le gaz à épurer contient souvent d'autres gaz qu'il est dans certains cas nécessaire d'éliminer en même temps que l'hydrogène sulfuré. C'est le cas notamment de l'anhydride carbonique contenu dans un gaz naturel devant être liquéfié.

La Demanderesse a mis au point un procédé permettant de résoudre les impératifs décrits ci-dessus.

Le but de la présente invention est donc l'élimination de l'hydrogène sulfuré contenu dans des mélanges gazeux.

A cet effet, l'invention a pour objet un procédé d'élimination de l'hydrogène sulfuré contenu dans un gaz à épurer, dans lequel on effectue la réaction dudit hydrogène sulfuré et d'anhydride sulfureux, dans un solvant de ces deux composés et en présence d'un excès d'hydrogène sulfuré, ladite réaction conduisant à l'obtention:
- d'une part, d'un effluent gazeux contenant l'excès d'hydrogène sulfuré utilisé pour la réaction,
- d'autre part, de soufre formé lors de la réaction,

et dans lequel ladite réaction est suivie d'une étape d'absorption de l'excès d'hydrogène sulfuré contenu dans l'effluent gazeux dans le même solvant que celui utilisé pour la réaction, ladite absorption conduisant à l'obtention:
- d'une part, d'un effluent gazeux,
- d'autre part, d'une solution d'hydrogène sulfuré dans le solvant, ladite solution étant recyclée au réacteur,

ledit procédé étant caractérisé en ce que la réaction est précédée d'une étape d'absorption, dans le même solvant, de l'hydrogène sulfuré contenu dans le gaz à épurer, ladite absorption conduisant à l'obtention:
- d'une part, d'un gaz épuré en hydrogène sulfuré,
- d'autre part, d'une solution d'hydrogène sulfuré dans le solvant, cette solution étant utilisée pour fournir l'hydrogène sulfuré nécessaire à la réaction de l'hydrogène sulfuré et de l'anhydride sulfureux.

Dans le procédé selon l'invention, l'étape d'absorption de l'hydrogène sulfuré contenu dans le gaz à épurer peut être effectuée à une température comprise entre 0 et 70°C, de préférence entre 20 et 60°C, et à une pression comprise entre 1 et 150 bars.

Si le gaz à épurer contient de l'anhydride carbonique, il est possible d'absorber l'anhydride carbonique, au moins en partie, à condition d'utiliser un taux de solvant suffisamment élevé pour absorber l'anhydride carbonique et l'hydrogène sulfuré contenus dans le gaz à épurer.

Le solvant utilisé pour l'absorption de l'hydrogène sulfuré (et éventuellement de l'anhydride carbonique) et pour la réaction de l'hydrogène sulfuré et de l'anhydride sulfureux peut comprendre au moins un composé choisi dans le groupe constitué par les glycols et les monoéthers de glycols, comme par exemple l'éthylène glycol, le diéthylène glycol, le triéthylène glycol, ainsi que les éthers monométhylique et monoéthylique des diéthylène- et triéthylène glycols. Les monoéthers de glycols sont préférés et, notamment, l'éther monométhylique du diéthylène glycol.

Dans le procédé selon l'invention, la réaction de l'anhydride sulfureux et de l'hydrogène sulfuré est effectuée en présence d'un excès d'hydrogène sulfuré, le rapport molaire $H_2S/SO_2$ étant, par exemple, compris entre 3 et 2, de préférence entre 2,2 et 2.

On obtient ainsi, à la suite de cette étape de réaction, un effluent gazeux pouvant contenir jusqu'à 5% en volume d'hydrogène sulfuré. L'excès d'hydrogène sulfuré contenu dans cet effluent est ensuite absorbé dans le même solvant que celui dans lequel est effectuée la réaction, la solution ainsi obtenue étant recyclée à l'étape de réaction.

La pression régnant à l'intérieur du réacteur peut être relativement basse. Il est préférable, toutefois, pour améliorer le contact entre les composés réagissant, d'établir une pression qui peut être comprise entre 1 et 150 bars, de préférence entre 5 et 80 bars.

La température régnant à l'intérieur du réacteur peut être telle que le soufre formé par la réaction de l'hydrogène sulfuré et de l'anhydride sulfureux soit liquide ou solide, c'est-à-dire que cette température peut être supérieure ou inférieure à 112°C.

La température du réacteur est, de préférence, inférieure à 112°C, de manière à recueillir le soufre formé à l'état solide, afin de ne pas avoir à refroidir le soufre en solution dans des échangeurs qui risquent de se trouver rapidement encrassés.

La température dans le réacteur est de préférence comprise entre 0 et 70°C.

La limite inférieure de cette gamme préférentielle est choisie en raison de la viscosité du solvant, qui devient importante aux basses températures.

La limite supérieure de cette gamme préférentielle est choisie de façon à ce que le soufre ne cristallise pas lors du refroidissement du solvant effectué avant que celui-ci ne soit conduit dans des tours d'absorption.

Le gaz partiellement épuré sortant du réacteur est conduit dans un absorbeur où règne une température qui doit être telle que l'hydrogène sulfuré soit suffisamment soluble dans le solvant et que la tension de vapeur du solvant utilisé ne soit pas trop élevée. Elle peut être comprise, par exemple, entre 0 et 150°C et, de préférence, entre 20 et 60°C.

La pression à l'intérieur de cet absorbeur est de préférence voisine de celle régnant à l'intérieur du réacteur.

Le débit du solvant dans l'absorbeur doit être tel que la quasi-totalité de l'hydrogène sulfuré qui est introduit soit dissous dans le solvant. Afin d'améliorer

l'absorption de l'excédent d'hydrogène sulfuré, le gaz partiellement épuré peut contenir jusqu'à 50% en volume et, de préférence, de 0,1 à 35 % en volume d'ammoniac.

La solution d'hydrogène sulfuré obtenue lors de l'étape d'absorption étant recyclée au réacteur, l'ammoniac qu'elle contient joue également un rôle de catalyseur de la réaction. En outre, l'ammoniac neutralise les sous-produits acides, tels que les acides polythioniques formés dans le réacteur.

Afin d'améliorer la solubilité de l'ammoniac dans le solvant, celui-ci peut contenir jusqu'à 75% en poids d'eau et, de préférence, jusqu'à 20% en poids d'eau. La limite supérieure de la teneur en eau du solvant est fixée par la nécessité de conserver au solvant un bon pouvoir solvant vis-à-vis de l'anhydride sulfureux; or ce pouvoir diminue lorsque la teneur en eau augmente, contrairement à ce qui est observé pour l'ammoniac. L'anhydride sulfureux nécessaire à la réaction peut être produit par la combustion du soufre formé lors de la réaction de l'hydrogène sulfuré et de l'anhydride sulfureux. Cette combustion peut être effectuée dans un four à soufre, avec de l'oxygène pur, ce qui permet d'introduire directement l'anhydride sulfureux dans le réacteur. Elle peut être également effectuée avec de l'air ou avec de l'air enrichi par de l'oxygène, l'anhydride sulfureux produit contenant alors de l'azote; dans ce cas, l'anhydride sulfureux est de préférence mis en solution dans le solvant préalablement à son introduction dans le réacteur; cette mise en solution peut être effectuée par absorption à une température comprise entre 0 et 150°C de préférence entre 20 et 60°C, et à une pression comprise entre 1 et 10 bars. Le débit de solvant doit être tel que la dissolution de l'anhydride sulfureux soit pratiquement totale.

La figure unique, jointe à la description, à titre d'illustration non limitative, est un schéma d'une unité

industrielle utilisant le procédé selon l'invention.

En référence à la figure, le gaz à épurer contenant un ou plusieurs hydrocarbures, de l'hydrogène sulfuré et de l'anhydride carbonique, est introduit par la ligne 1 dans la partie inférieure d'une tour d'absorption 2.

La tour d'absorption 2 est alimentée dans sa partie supérieure, par la ligne 3, en un solvant dont la provenance sera expliquée plus loin. Dans la tour d'absorption, l'hydrogène sulfuré est pratiquement dissous en totalité dans le solvant. La solution d'hydrogène sulfuré est recueillie au fond de la tour 2 par la ligne 4. On recueille au sommet de la tour 2, par la ligne 5, le gaz épuré.

La ligne d'alimentation 1 peut être équipée d'un compresseur 6, si la pression du gaz à épurer n'est pas suffisante.

Selon que l'on désire ou non que le gaz épuré recueilli par la ligne 5 contienne ou non, ou tout au moins très peu, d'anhydride carbonique, on ajuste le débit de solvant introduit par la ligne 3.

Plus le débit du solvant sera important, moins le gaz épuré de la ligne 5 contiendra d'anhydride carbonique.

La solution d'hydrogène sulfuré dans le solvant, recueillie par la ligne 4, contenant plus ou moins d'anhydride carbonique, est conduite dans la partie inférieure d'un réacteur 6'.

Au préalable, cette solution a été détendue à une pression intermédiaire entre la pression de la tour 2 et celle du réacteur 6' dans une enceinte de détente 60; le gaz de détente est recyclé à la tour 2 par la ligne 61, la solution recueillie au fond de l'enceinte 60, par la ligne 62, étant conduite au réacteur 6'. Cette opération vise à

minimiser la quantité d'hydrocarbures entraînés par la ligne 4 par dissolution dans le solvant.

Le réacteur 6' est également alimenté dans sa partie inférieure par la ligne 7, par une solution d'anhydride sulfureux dans le solvant, dont la provenance sera expliquée plus loin. Le débit de la solution d'anhydride sulfureux amenée par la ligne 7 est tel qu'il correspond à une mole d'anhydride sulfureux pour deux moles d'hydrogène sulfuré contenu dans la solution introduite dans le réacteur 6' par la ligne 4.

On introduit également dans le réacteur 6', par la ligne 8, du solvant contenant en solution de l'hydrogène sulfuré en quantité telle que le rapport molaire $H_2S/SO_2$ dans le réacteur soit compris entre 2 et 3, de telle façon que la réaction entre l'hydrogène sulfuré et l'anhydride sulfureux soit effectuée en présence d'un excès d'hydrogène sulfuré. La solution d'hydrogène sulfuré introduite par la ligne 8 dans le réacteur 6', contient également de l'ammoniac. La provenance de la solution de la ligne 8 sera expliquée plus loin.

La température à l'intérieur du réacteur peut être inférieure ou supérieure à 112°C, c'est-à-dire à la température de fusion du soufre. Le soufre formé par la réaction de l'hydrogène sulfuré et de l'anhydride sulfureux peut donc se trouver à l'état solide ou liquide.

Dans l'exemple de mise en oeuvre du procédé selon l'invention, la température à l'intérieur du réacteur est inférieure à 112°C.

Le soufre formé lors de la réaction de l'hydrogène sulfuré et de l'anhydride sulfureux se trouve donc à l'état cristallisé en suspension dans le solvant.

Le réacteur 6' est de préférence du type agité et est donc équipé d'un agitateur 9.

Le réacteur préférentiel utilisé dans le procédé selon l'invention fait l'objet d'une demande de brevet déposée le même jour que la présente demande.

L'agitation dans le réacteur développe une plus grande aire interfaciale par unité de volume et assure un coefficient global de transfert plus élevé qu'un réacteur garni. Il en résulte les avantages suivants:

- il est possible d'utiliser un réacteur de volume plus petit;
- les réactions dont la cinétique est conditionnée par le transfert gaz-liquide, notamment la réaction de Claus sont favorisées, aux dépens de certaines réactions secondaires qui se développent dans un réacteur de volume important, c'est-à-dire les réactions entre les oléfines, l'hydrogène sulfuré et le soufre, avec pour conséquence une formation moindre de sous-produits;
- on évite les bouchages, provoqués par les sels d'ammonium produits ou les produits de corrosion, par effet de filtre d'un garnissage;
- la réaction de Claus peut être mise en oeuvre à froid et avec, par conséquent, du soufre cristallisé présent dans le réacteur;
- le coefficient de transfert de chaleur entre le milieu du réacteur et la paroi de celui-ci est élevé, ce qui permet d'évacuer les calories de la réaction par une double enveloppe dans laquelle circule de l'eau, au lieu d'avoir à utiliser un échangeur réfrigérant interne, qui serait sujet aux bouchages;
- la décantation du soufre et des autres sous-produits solides éventuellement présents est évitée, avec pour résultat une simplification des évacuations, une seule sortie par le haut du réacteur pouvant être prévue pour toutes les phases: gaz, liquide, solide.

On peut remarquer sur la figure que les différentes injections dans le réacteur se font de façon étagée.

La solution d'anhydride sulfureux dans le solvant est injectée par la ligne 7, à un niveau supérieur à celui où la solution d'hydrogène sulfuré est injectée par la ligne 62.

La solution d'anhydride sulfureux est injectée à l'aide d'un diffuseur non représenté, mais décrit dans la demande de brevet précitée concernant le réacteur.

On évite ainsi un rapport $\frac{SO_2}{H_2S}$ localement trop élevé, qui favorise la formation d'acides polythiomiques.

L'ammoniac est enfin injecté dans la solution contenue dans la ligne 8, après que la plus grande partie de la conversion de l'anhydride sulfureux en soufre ait été effectuée, ce qui peut se faire en dehors de la présence d'ammoniac agissant comme catalyseur.

La conversion totale de l'anhydride sulfureux est effectuée en haut du réacteur en présence d'ammoniac, ce qui a pour effet de minimiser la formation de thiosulfate d'ammonium, favorisée par la présence d'ammoniac.

L'effluent recueilli à la sortie du réacteur par le conduit 11 est conduit dans un décanteur 12.

On recueille au sommet du décanteur 12 un effluent gazeux, constitué par des hydrocarbures, de l'eau, de l'hydrogène sulfuré, de l'ammoniac et éventuellement de l'anhydride carbonique, si le débit de solvant dans la tour 2 est trop faible pour que la quasi-totalité de ce gaz y soit absorbée. Cet effluent gazeux se sépare d'une suspension de soufre dans le solvant, contenant également à l'état dissous l'eau formée lors de la réaction, des hydrocarbures, de l'hydrogène sulfuré et de l'ammoniac et, éventuellement, une faible quantité d'anhydride carbonique,

0102300

qui ne se sépare pas avec l'effluent gazeux.

Dans le décanteur, la suspension se sépare en deux phases:

- une phase supérieure 13 composée de solvant contenant en solution de l'eau, des hydrocarbures, de l'hydrogène sulfuré et de l'ammoniac,
- une phase inférieure 14 composée de soufre cristallisé imbibé de solvant.

L'effluent gazeux recueilli par la ligne 10 est conduit par l'intermédiaire de la ligne 15 dans une tour d'absorption 16.

La tour d'absorption 16 est alimentée en solvant par la ligne 17, solvant dont la provenance sera expliquée plus loin.

La tour d'absorption 16 est également équipée d'une ligne 18 destinée à alimenter l'unité en ammoniac au démarrage de l'unité et à introduire éventuellement des appoints d'ammoniac quand c'est nécessaire.

On évacue au sommet de la tour 16, par la ligne 19, l'anhydride carbonique extrait dans la tour 2.

On recueille, au fond de la tour 16, par la ligne 8, une phase liquide constituée par du solvant contenant en solution de l'hydrogène sulfuré, de l'ammoniac et de l'eau. Cette phase est recyclée au réacteur 6'.

La phase supérieure 13 recueillie au sommet du décanteur 12 par la ligne 20 est conduite, après passage dans un réchauffeur 21, dans une colonne d'entraînement 22 équipée d'un rebouilleur 23 et d'un condenseur 24. Dans cette colonne, qui fonctionne à une température comprise entre 100 et 145°C en tête de la colonne et 145 et 200°C

au fond, selon la pression de fonctionnement, laquelle peut être comprise entre 1 et 4 bars, l'eau contenue dans la charge de la ligne 20 sert à entraîner les hydrocarbures, l'hydrogène sulfuré et l'ammoniac contenus dans la charge de la ligne 20.

On recueille ainsi en fond de la colonne 22, par la ligne 25, le solvant, qui, après passage dans un réfrigérant 26, est conduit dans un réservoir 27.

Le solvant nécessaire à l'alimentation de la tour d'absorption 16 est prélevé dans le réservoir 27 par la ligne 17.

On recueille au sommet de la colonne 22, par la ligne 28, un mélange d'hydrocarbures, d'hydrogène sulfuré, d'ammoniac et d'eau, qui est conduit dans une colonne d'entraînement à la vapeur d'eau 29 équipée d'un reflux 30. De la vapeur d'eau est introduite dans la colonne 29 par la ligne 31.

Cette colonne fonctionne à une température comprise entre 100 et 145°C et à une pression comprise entre 1 et 4 bars.

On recueille au fond de la colonne 29, par la ligne 32, de l'eau et, au sommet, par la ligne 33, un mélange d'hydrogène sulfuré, d'hydrocarbures et d'ammoniac, qui, après passage dans un compresseur 34, est recyclé à la colonne 16 par l'intermédiaire de la ligne 15.

La phase inférieure 14 contenue dans le décanteur 12 est recueillie par la ligne 35 et est conduite dans un réchauffeur 36, destiné à porter le soufre à l'état liquide. Il se produit alors un dégazage de gaz dissous, de l'hydrogène sulfuré, de l'ammoniac et des hydrocarbures, qui sont conduits à la colonne 22 par la ligne 57. Le soufre

liquide est conduit dans un filtre 37 destiné à séparer le sulfate d'ammonium formé par réaction de l'ammoniac et de l'anhydride sulfureux. Le sulfate d'ammonium est soutiré du filtre par la ligne 38.

On recueille à la sortie du filtre 37, par la ligne 39, du soufre liquide contenant du solvant, qui est conduit dans un séparateur liquide-liquide 40.

La phase liquide supérieure 41 est composée de solvant, qui est recyclé au réacteur 6' par la ligne 42.

La phase liquide inférieure 43 est composée de soufre liquide, qui est conduit dans un bac à soufre 44 par la ligne 45.

Le soufre est soutiré du bac 44 par la ligne 46. Une partie du soufre est évacuée par la ligne 47. Une autre partie du soufre sert à alimenter par la ligne 48 un four à soufre 49, qui est alimenté en air par la ligne 50. Afin de minimiser la quantité d'anhydride sulfurique $SO_3$ formé dans le four à soufre, celui-ci fonctionne avec un excès de soufre par rapport à l'air. Après condensation à la sortie du four dans un condenseur non représenté, l'excès de soufre est recyclé à la ligne 48 par la ligne 58.

Le mélange d'anhydride sulfureux et d'azote recueilli à la sortie du four 49 par la ligne 51 est conduit, après passage dans un réfrigérant 52, dans la partie inférieure d'une tour d'absorption 53.

On recueille dans le fond de la tour 53, par la ligne 7, une solution d'anhydride sulfureux dans le solvant, solution qui est conduite dans le réacteur 6'.

L'azote introduit dans la tour 53 par la ligne 51 est évacué au sommet de la tour par la ligne 55.

Le solvant nécessaire à l'alimentation de la tour 2 est prélevé dans le réservoir 27.

Le procédé selon l'invention peut être appliqué à l'élimination de l'hydrogène sulfuré contenu dans des gaz à épurer, quelle que soit la concentration dudit hydrogène sulfuré. Ces gaz à épurer peuvent être constitués, par exemple, par les effluents d'unités de raffinage de pétrole brut, comme les unités de désulfuration ou de craquage catalytique. Il peut s'agir également de gaz naturel, ou d'un effluent de gazéification de charbon.

Le procédé selon l'invention permet d'obtenir un gaz pouvant contenir moins de 5 ppm en volume d'hydrogène sulfuré.

L'exemple qui suit constitue une illustration de l'invention, sans pour autant la limiter.

EXEMPLE

Cet exemple concerne le traitement, par une unité telle que représentée sur la figure annexée, d'un gaz naturel.

La composition de ce gaz est donnée dans le Tableau I ci-après.

TABLEAU I

| Constituants | % en volume |
|---|---|
| Hydrogène sulfuré | 5 |
| Anhydride carbonique | 5 |
| Hydrocarbures | 90 |

Cet exemple décrit deux essais A et B de traitement de ce gaz.

L'essai A a été effectué dans des conditions dans lesquelles l'anhydride carbonique n'est que partiellement extrait dans la tour 2.

L'essai B a été effectué dans des conditions dans lesquelles l'anhydride carbonique est extrait en quasi-totalité dans la tour 2.

Le solvant utilisé lors de ces essais est l'éther monométhylique du diéthylène glycol.

Le réacteur 6 est un réacteur agité, les tours 2, 16 et 53 étant des colonnes à plateaux.

Les conditions de température et de pression pour les essais A et B dans les tours 2, 16, 53 et dans le réacteur 6' sont données dans le Tableau II ci-après:

TABLEAU II

| | | |
|---|---|---|
| Tour 2 | Température en °C | 40 |
| | Pression en bars | 50 |
| Tour 16 | Température en °C | 50 |
| | Pression en bars | 10 |
| Tour 53 | Température en °C | 50 |
| | Pression en bars | 1 |
| Réacteur 6' | Température en °C | 50 |
| | Pression en bars | 10 |

Les débits et compositions des principales lignes de l'unité sont indiqués dans le Tableau III ci-après.

TABLEAU III

| Ligne | | Essai | Composition | Débit en t/h |
|---|---|---|---|---|
| 1 | | A | Voir Tableau I | 8,2 |
| | | B | Voir Tableau I | 8,2 |
| 3 | | A | Solvant | 21 |
| | | B | - id - | 107 |
| 5 | EFFLUENT GAZEUX | A | Hydrocarbures: 96,4% en volume<br>Anhydride carbonique: 3,6 -<br>Hydrogène sulfuré 4 p.p.m | 7,1 |
| | | B | Hydrocarbures: ≃ 100% en volume<br>Anhydride carbonique: 100 p.p.m | 6,4 |
| 4 | EFFLUENT LIQUIDE | A | Hydrogène sulfuré: 3,4% en poids<br>Anhydride carbonique: 1,4% "<br>(le reste étant du solvant) | 22,1 |
| | | B | Hydrogène sulfuré: 0,7% en poids<br>Anhydride carbonique: 0,9% "<br>(le reste étant du solvant) | 108,7 |
| 7 | | A | Solvant contenant 4,2% en poids d'anhydride sulfureux | 16,9 |
| | | B | - id - | 16,9 |
| 19 | EFFLUENT GAZEUX | A | -Anhydride Carbonique: 99,5% en volume<br>-Hydrocarbures 0,3%<br>-Hydrogène sulfuré 2000 p.p.m -<br>-Ammoniac 500 - - | 0,3 |
| | | B | -Anhydride Carbonique: 98,3% en volume<br>-Hydrocarbures 1,5% -<br>-Hydrogène sulfuré 2000 p.p.m -<br>-Ammoniac 500 - - | 1,0 |

TABLEAU III

| Ligne | Essai | Composition | Débit en t/h |
|---|---|---|---|
| 17 | A | Solvant | 7 |
| | B | Solvant | 22 |
| 46 | A | Soufre | 1069 |
| | B | Soufre | 1068 |
| 47 | A | Soufre | 713 |
| | B | Soufre | 712 |
| 48 | A | Soufre | 356 |
| | B | Soufre | 356 |

La conversion de l'hydrogène sulfuré en soufre est supérieure dans les deux cas à 99%, ce qui montre bien l'intérêt du procédé selon l'invention.

Revendications

1. Procédé d'élimination de l'hydrogène sulfuré contenu dans un gaz à épurer, dans lequel on effectue la réaction dudit hydrogène sulfuré et d'anhydride sulfureux, dans un solvant de ces deux composés et en présence d'un excès d'hydrogène sulfuré, ladite réaction conduisant à l'obtention:

- d'une part, d'un effluent gazeux contenant l'excès d'hydrogène sulfuré utilisé pour la réaction,
- d'autre part, de soufre formé lors de la réaction,

et dans lequel ladite réaction est suivie d'une étape d'absorption de l'excès d'hydrogène sulfuré contenu dans l'effluent gazeux dans le même solvant que celui utilisé pour la réaction, ladite absorption conduisant à l'obtention:

- d'une part, d'un effluent gazeux,
- d'autre part, d'une solution d'hydrogène sulfuré dans le solvant, ladite solution étant recyclée à l'étape de réaction,

ledit procédé étant caractérisé en ce que ladite réaction est précédée d'une étape d'absorption de l'hydrogène sulfuré contenu dans le gaz à épurer dans le même solvant que celui utilisé pour la réaction, ladite absorption conduisant à l'obtention:

- d'une part, d'un gaz épuré en hydrogène sulfuré,
- d'autre part, d'une solution d'hydrogène sulfuré dans le solvant,

cette solution étant utilisée pour fournir l'hydrogène sulfuré nécessaire à l'étape de réaction de l'hydrogène sulfuré et de l'anhydride sulfureux.

2. Procédé selon la revendication 1, caractérisé en ce que le gaz à épurer contient de l'anhydride carbonique, qui est absorbé au moins en partie lors de l'absorption de l'hydrogène sulfuré contenu dans le gaz à épurer.

3. Procédé selon les revendications 1 et 2, caractérisé en ce que, lors de la réaction de l'hydrogène sulfuré et de

l'anhydride sulfureux, le rapport molaire $H_2S/SO_2$ est compris entre 3 et 2 et de préférence entre 2,2 et 2.

4. Procédé selon l'une des revendications 1 à 3, caractérisé en ce que le solvant utilisé pour l'étape de réaction et les étapes d'absorption comprend au moins un composé choisi dans le groupe constitué par les glycols et les monoéthers de glycols.

5. Procédé selon l'une des revendications 1 à 4, caractérisé en ce que le solvant comprend au moins un composé choisi dans le groupe constitué par l'éthylène glycol, le diéthylène glycol, le triéthylène glycol et les éthers monométhylique et monoéthylique des diéthylène et triéthylène glycol.

6. Procédé selon l'une des revendications 1 à 5, caractérisé en ce que le solvant comprend l'éther monométhylique du diéthylène glycol.

7. Procédé selon l'une des revendications 1 à 6, caractérisé en ce que la réaction et l'absorption consécutive de l'excès d'hydrogène sulfuré contenu dans le gaz partiellement épuré sont conduites en présence d'ammoniac, en quantité telle que le gaz partiellement épuré soumis à l'absorption contient jusqu'à 50% en volume et, de préférence, de 0,1 à 35% en volume d'ammoniac.

8. Procédé selon la revendication 7, caractérisé en ce que le solvant contient au plus 75% et, de préférence, au plus 20% en poids d'eau.

9. Procédé selon l'une des revendications 1 à 8, caractérisé en ce que la réaction est effectuée à une température inférieure à 112°C, de préférence entre 0 et 70°C, et à une pression comprise entre 1 et 150 bars, de préférence entre 5 et 80 bars.

10. Procédé selon l'une des revendications 1 à 6, caractérisé en ce que la réaction entre l'hydrogène sulfuré et l'anhydride sulfureux est conduite dans un réacteur où la solution d'hydrogène sulfuré est injectée à un niveau inférieur à celui auquel est introduite la solution d'anhydride sulfureux.

11. Procédé selon les revendications 7 et 10, en combinaison, caractérisé en ce que l'ammoniac est introduit dans le réacteur avec une quantité additionnelle de solution d'hydrogène sulfuré de recycle à un niveau supérieur à celui où l'anhydride sulfureux est injecté dans le réacteur.

1/1

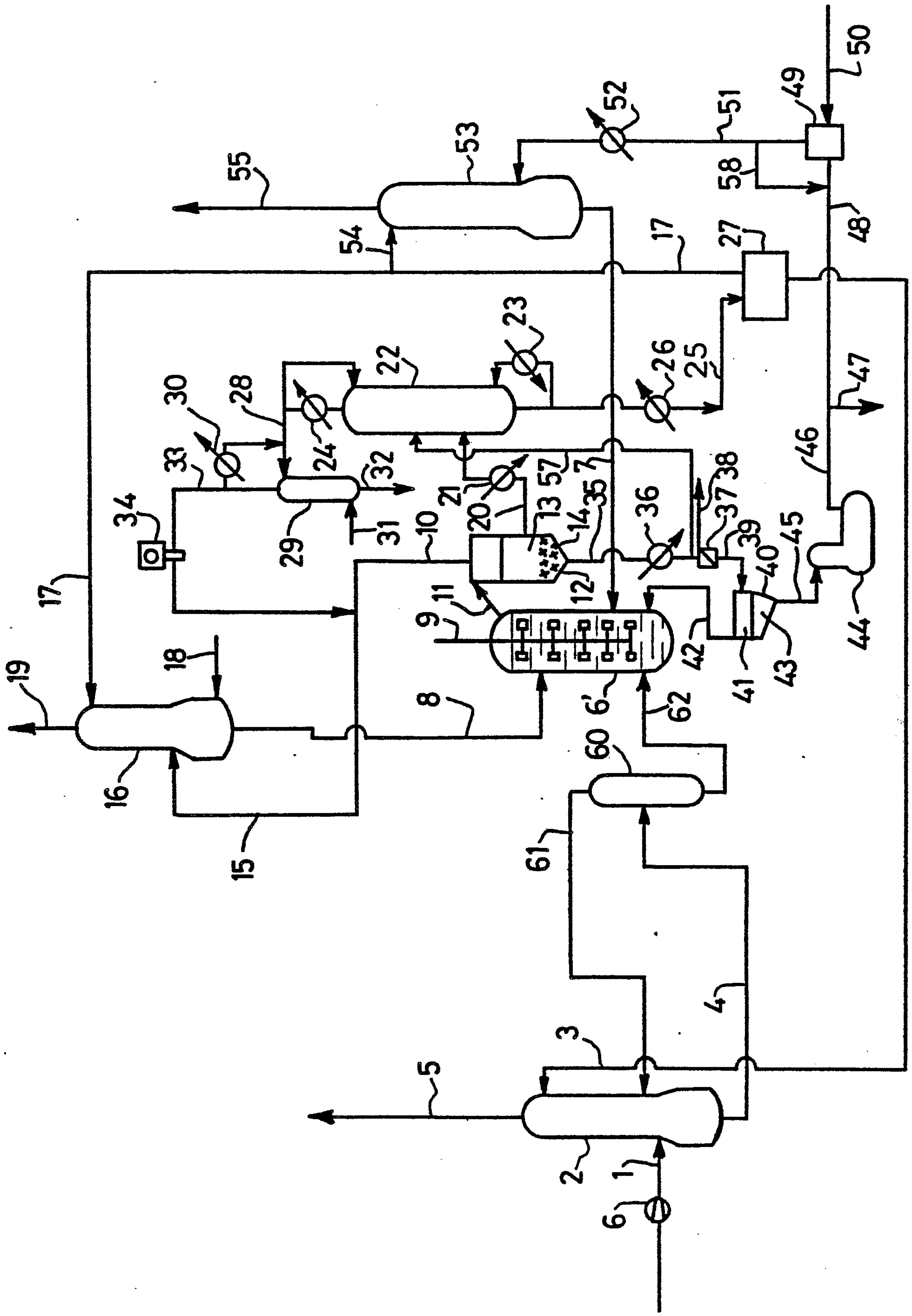

0102300

Office européen des brevets

# RAPPORT DE RECHERCHE EUROPEENNE

Numéro de la demande

EP 83 40 1704

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | US-A-3 953 586 (W. TANIMURA)<br>* Colonne 2, ligne 12 - colonne 6, ligne 61; figure * | 1,4,5,9 | C 01 B 17/05<br>B 01 D 53/34<br>C 10 K 1/16 |
| Y | FR-A-2 336 163 (COMP. FRANCAISE DE RAFFINAGE)<br>* Page 18, lignes 1-18 * | 1 | |
| D,Y | FR-A-2 411 802 (COMP. FRANCAISE DE RAFFINAGE)<br>* Page 6, lignes 23-35; page 11, ligne 1 - page 12, ligne 26; figure * | 1,3-9,11 | |

DOMAINES TECHNIQUES RECHERCHES (Int. Cl. 3)

C 01 B
B 01 D
C 10 K

Le présent rapport de recherche a été établi pour toutes les revendications

| Lieu de la recherche | Date d'achèvement de la recherche | Examinateur |
|---|---|---|
| LA HAYE | 25-11-1983 | PYFFEROEN K. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant